(19) Europäisches Patentamt
European Patent Office
Office européen des brevets

(11) Veröffentlichungsnummer: **0 170 180**
**A1**

(12) **EUROPÄISCHE PATENTANMELDUNG**

(21) Anmeldenummer: 85109112.4

(22) Anmeldetag: 20.07.85

(51) Int. Cl.⁴: C 07 C 129/12
A 61 K 31/245

(30) Priorität: 03.08.84 DE 3429114

(43) Veröffentlichungstag der Anmeldung: 05.02.86 Patentblatt 86/6

(84) Benannte Vertragsstaaten: AT BE CH DE FR GB IT LI LU NL SE

(71) Anmelder: SCHERING AKTIENGESELLSCHAFT Berlin und Bergkamen
Müllerstrasse 170/178 Postfach 65 03 11
D-1000 Berlin 65(DE)

(72) Erfinder: Habenicht, Ursula, Dr.
Westfälische Strasse 62
D-1000 Berlin 31(DE)

(72) Erfinder: Kirsch, Gerald, Dr.
Luciusstrasse 6 B
D-1000 Berlin 33(DE)

(72) Erfinder: Klose, Walter, Dr.
Beatestrasse 9 G
D-1000 Berlin 27(DE)

(72) Erfinder: Laurent, Henry, Dr.
Glambecker Weg 21
D-1000 Berlin 28(DE)

(72) Erfinder: El Etreby, Fathy, Prof. Dr.
Dachsberg 7 B
D-1000 Berlin 33(DE)

(54) **Substituierte Guanidinobenzoesäurephenylester, Verfahren zu ihrer Herstellung und ihre Verwendung als Arzneimittel.**

(57) Es werden Guanidinobenzoesäureester der allgemeinen Formel I

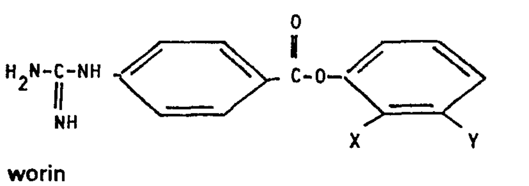

(I),

worin
X für ein Wasserstoffatom, einen Cyanrest oder eine Alkoxygruppe mit 1 - 4 Kohlenstoffatomen und
Y für ein Wasserstoffatom, eine Alkoxy- oder Alkoxycarbonylgruppe mit 1 - 4 Kohlenstoffatomen oder eine

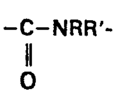

Gruppe mit R und R' in der Bedeutung eines Wasserstoffatoms oder eines niederen gesättigten Alkylrestes mit 1 - 4 Kohlenstoffatomen steht, wobei X und Y nicht gleichzeitig Wasserstoff sein sollen, sowie deren physiologisch unbedenkliche Salze mit anorganischen oder organischen Säuren beschrieben.

Die neuen Verbindungen weisen eine signifikant stärkere Wirksamkeit gegenüber den bekannten Aryl-Guanidinobenzoaten sowohl als (Human)-Akrosin-Inhibitor wie auch als Kontrazeptiva im Tierversuch auf.

EP 0 170 180 A1

Die Erfindung betrifft neue substituierte Guanidinobenzoesäurephenylester, ihre Verwendung sowie Verfahren zu ihrer Herstellung und diese enthaltende pharmazeutische Präparate gemäß den Patentansprüchen.

Arylester von 4-Guanidinobenzoesäuren sind für eine vaginale Kontrazeption geeignet (US-Patent 4,423,069). Sie wirken als Inhibitor des für eine Befruchtung essentiellen Enzyms Akrosin, einer innerhalb des Akrosoms der Spermatozoen lokalisierten Serin-Proteinase. Akrosin ist entscheidend wichtig für den Teil der Befruchtung, bei dem die Spermatozoen die Zona pellucida (Oolemma), die innerste der drei Schichten, die die Eizelle umgeben, durchdringen.

Es wurde nun gefunden, daß die neuen Guanidinobenzoesäureester der allgemeinen Formel I

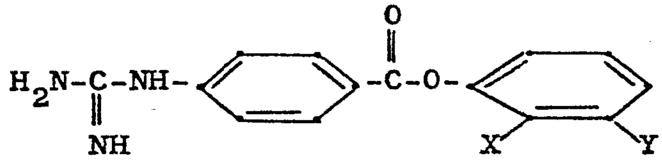

(I),

worin X für ein Wasserstoffatom, einen Cyanrest oder eine Alkoxygruppe mit 1 - 4 Kohlenstoffatomen, wie beispielsweise die Methoxy-, Ethoxy- oder Propoxygruppe, und Y für ein Wasserstoffatom, eine Alkoxy- oder Alkoxycarbonylgruppe mit 1 - 4 Kohlenstoffatomen, wie beispielsweise die Methoxy-, Ethoxy-, Propoxy- oder Methoxycarbonyl-, Ethoxycarbonyl-, Propoxycarbonylgruppe, oder eine $-\underset{\underset{O}{\|}}{C}-NRR'$-Gruppe mit R und R' in der Bedeutung eines Wasserstoffatoms oder eines niederen gesättigten Alkylrestes mit 1 - 4 Kohlenstoffatomen, wie beispielsweise die Methyl-, Ethyl-, Propyl- oder i-Propylgruppe, wobei X und Y nicht gleichzeitig Wasserstoff sein sollen, steht sowie deren physiologisch unbedenkliche Salze mit anorganischen oder organischen Säuren, wie beispielsweise Salzsäure, Citronensäure oder p-Toluolsulfonsäure, überraschenderweise eine signifikant stärkere Wirksamkeit gegenüber den bekannten Aryl-Guanidinobenzoaten sowohl als (Human)-Akrosin-Inhibitor wie auch als Kontrazeptiva im Tierversuch aufweisen.

## Pharmakologische Beobachtungen

### 1.) Akrosin (-Human)-Assay

Dieser Test stellt das eigentliche Primärscreening dar. Er gibt einen Anhaltspunkt über die inhibitorische Effektivität einer Verbindung am Enzym (Humanakrosin) selbst, erlaubt aber noch keine Aussage über die biologische Effektivität (d.h. Hemmung von Akrosin im intakten Spermium sowie kontrazeptive Wirksamkeit) der untersuchten Verbindungen.

Das durch eine saure Extraktion aus Humansperma gewonnene Enzym Akrosin wird mit verschiedenen Konzentrationen des potentiellen Inhibitors inkubiert, und es wird diejenige Konzentration bestimmt, die zu einer 50 %igen Hemmung der Akrosinaktivität führt. Die Serum-Proteinase Akrosin spaltet physiologischerweise bevorzugt Arginin- und Lysin-Gruppen. Deshalb wird für die in vitro-Untersuchung zur Aktivitätsbestimmung von

Akrosin ein Benzoyl-Arginin-Äthylester als Substrat verwendet.

Die gemessenen Daten (Tabelle 1) belegen nicht nur eine inhibitierende Wirkung der erfindungsgemäßen Verbindungen (Nr. 2 bis 6) auf das Enzym Akrosin, sie belegen auch, daß deren Effektivität größer ist als die der als Lead-Verbindung anzusehenden vorbekannten Substanz Nr. 1.

2.) Gelatinolyse

Dieser Test ist ebenfalls als Bestandteil des Primärscreenings anzusehen. Für die erwünschte kontrazeptive Wirkung eines Akrosininhibitors ist es - neben der eigentlichen Akrosinhemmung am isolierten Enzym - von essentieller Bedeutung, daß ein solcher Inhibitor in der Lage ist, das Plasmalemm und die äußere akrosomale Membran eines intakten Spermiums zu durchdringen, da Akrosin an der inneren akrosomalen Membran lokalisiert ist.

Als besonders geeignet für die Untersuchung dieser speziellen Fragestellung hat sich das Gelatinolyse-Modell nach P. Gaddum und R.Z. Blandau (Science 170: 749 - 751), modifiziert nach W.B. Schill (Int. J. Andrology 4: 25 - 38) und nach eigenen Ausarbeitungen erwiesen. Bei dieser Methode wird mit unbelichteten, fixierten und mit Methylenblau gefärbten Filmplatten für die Autoradiographie gearbeitet, deren Gelatineschicht durch die lytische Aktivität von Akrosin aufgelöst wird. Als Folge bilden sich um die Spermienköpfe gut sichtbare, weite Höfe (sog. Halo-Bildung/Lysishof). Bei einer Hemmung von Akrosin unterbleibt die Ausbildung dieser Höfe.

Um die Penetrierfähigkeit eines erwiesenen Akrosininhibitors und damit die Hemmung von Akrosin am intakten Spermium zu untersuchen, wird Humansperma mit mehreren Konzentrationen des Inhibitors präinkubiert. Anschließend wird ein Aliquot auf die entsprechend vorbereiteten (s.o.), auf Objektträger aufgezogenen Gelatineplättchen appliziert und im Brutschrank in feuchter Atmosphäre inkubiert. Anschließend wird unter dem Phasenkontrastmikroskop der Grad der Halo-Bildung semiquantitativ bestimmt.

Die in Tabelle 2 angegebenen pharmakologischen Ergebnisse dieses Testes belegen stellvertretend für die beanspruchten Verbindungen die Effektivität bzw. Überlegenheit der angeführten Substanzen über die vorbekannte Lead-Verbindung (Nr. 1).

3.) <u>In vitro-Fertilisation (Maus)</u>

Dieser Test erlaubt eine erste Überprüfung der kontrazeptiven Effektivität sowie eine Überprüfung des Wirkprinzips per se:

In einem Mediumtropfen nach Brinster, Whitten und Whittingham, der den Inhibitor enthält, werden zunächst die durch Superovulation gewonnenen Oocyten überführt. Anschließend wird ein Aliquot des unbehandelten Maussperma hinzupipettiert. Die Konzentrationen des Inhibitors werden so gewählt, daß keine Hemmung der Motilität vorliegt. 24 Stunden später wird die Anzahl der geteilten und nicht geteilten Oocyten und damit die Fertilitätsrate bestimmt.

Es wird also überprüft, ob der zu untersuchende Akrosininhibitor, appliziert in einem System, in dem es zum

unmittelbaren Kontakt zwischen Oocyte und Spermium kommt, eine Fertilisation hemmen kann - ohne daß eine Hemmung der Motilität der Spermien vorliegt.

In Tabelle 3 sind die auf Grund dieses Tests bestimmten fertilitätshemmenden Wirkungen der erfindungsgemäßen Substanzen am Beispiel der 2-Cyano- bzw. 3-Methoxycarbonyl-Verbindung (Nr. 2 bzw. 3) im Vergleich zur vorbekannten Lead-Substanz (Nr. 1) aufgeführt: Die Werte zeigen eine deutliche Überlegenheit der neuen Substanzen auf.

4.) In-vivo-Fertilisation (Kaninchen) nach artifizieller Inseminierung präinkubierter Spermien

Dieser Test ermöglicht Aussagen zur kontrazeptiven Effektivität unter in-vivo-Bedingungen:

Kaninchensperma, das mit Hilfe einer künstlichen Vagina gewonnen wird, wird mit dem zu untersuchenden Akrosininhibitor präinkubiert. Anschließend werden zur Induktion einer Superovulation mit PMS (pregnant mare serum) vorbehandelte Kaninchenweibchen mit dem vorbehandelten Sperma artifiziell inseminiert. Zur Auslösung der Superovulation erhalten sie abschließend HCG (human chorionic gonadotropin).

36 Stunden später werden die Tiere getötet, die Ovidukte und Uteri entnommen und gespült, um die frühen Embryonen bzw. ungeteilten Oocyten zu gewinnen und anhand deren Anzahl die Fertilisationsrate zu bestimmen.

Auch die Ergebnisse (s. Tabelle 4) dieses in-vivo-Testes belegen für die erfindungsgemäßen Verbindungen, aufgezeigt am Beispiel der Verbindungen Nr. 2 und 3, im Vergleich zur Lead-Substanz (Nr. 1) eine überraschend hohe Steigerung der fertilitätshemmenden Wirkung (die Fertilitätsrate sinkt von 51 auf 4 bzw. 1 %).

Für die praktische Anwendung der erfindungsgemäßen Verbindungen als Arzneimittel an Säugetieren, einschließlich Menschen, werden sie nach an sich bekannten Methoden der Galenik zu vaginalen Kontrazeptiva formuliert.

Als Applikationsform sind Gele, Cremes, Schaum, Zäpfchen oder lösliche Filme geeignet. Bevorzugt ist die Anwendung eines "slow releasing"-Systems, damit ein über längere Zeit (bis 24 Stunden) anhaltender kontrazeptiver Effekt gewährleistet wird.

Als zweckmäßige Trägermaterialien, die allein oder zusammen mit geeigneten Lösungsmitteln wie Wasser benutzt werden können und eine leichte Verteilung des Wirkstoffes gewährleisten müssen, selbst aber nicht zu flüssig sein dürfen, kommen u.a. Polyäthylenglykol-Derivate sowie Methyl-Zellulose-Verbindungen infrage.

Die Konzentration des Wirkstoffes liegt im Bereich von 0,1 - 10,0 Gewichtsprozent.

Für die Anwendung am Menschen beträgt die Dosiseinheit pro Anwendung 1 - 10 mg.

Die Herstellung der erfindungsgemäßen Guanidinobenzoesäureester der allgemeinen Formel I erfolgt gemäß Anspruch 7 nach an sich bekannten Methoden für die Veresterung von Carbonsäuren. Bevorzugt ist die bei 0 °C - 30 °C durchgeführte, durch Dicyclohexylcarbodiimid bewirkte und einen sauren, wie z.B. p-Toluolsulfonsäure, und einen basischen, wie z.B. Pyridin, Katalysator beschleunigte Kondensation von Phenolen der allgemeinen Formel II

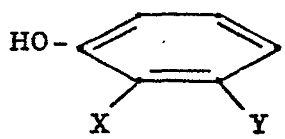

wobei X und Y die in Anspruch 1 genannte Bedeutung haben, mit einem Salz der 4-Guanidinobenzoesäure (vorzugsweise das Hydrochlorid) in einem geeigneten inerten aprotischen, polaren Lösungsmittel, wie beispielsweise Dimethylformamid, Dimethylacetamid, Dimethylsulfoxid, Tetrahydrofuran, Diethylether, Methylenchlorid, Acetonitril oder Pyridin. Nach geeigneter Aufarbeitung und Reinigung (als wichtigstes Nebenprodukt muß N,N'-Dicyclohexylharnstoff entfernt werden) durch konventionelle Techniken wie Filtration, Extraktion mit geeigneten Lösungsmitteln, sowie vor allem chromatographische Trennmethoden werden die gewünschten Guanidiobenzoesäureester der allgemeinen Formel I, je nach Reaktionsführung, als freie Basen oder als anorganische oder organische Salze, wie z.B. Hydrochloride, Citrate oder p-Toluolsulfonate, erhalten. Durch dem Fachmann geläufige Art und Weise können die Salze in die freien Basen bzw. die freien Basen in die Salze umgewandelt werden.

## T a b e l l e I

Bestimmung der $I_{50}$ verschiedener
Aryl-4-Guanidinobenzoatderivate für Humanakrosin

| Verbindung | Substrat: Benzoyl-L-arginyleth.-ester |
|---|---|
| | $I_{50}$ $10^{-9}$ [M] |
| 1.) 4-Guanidinobenzoesäure-(2-methoxycarbonylphenyl)-ester Hydrochlorid | 733 |
| 2.) 4-Guanidinobenzoesäure-(2-cyanophenyl)-ester Hydrochlorid | 40 |
| 3.) 4-Guanidinobenzoesäure-(3-ethoxycarbonylphenyl)-ester Hydrochlorid | 200 |
| 4.) 4-Guanidinobenzoesäure-(2,3-dimethoxyphenyl)-ester Hydrochlorid | 400<br>500 |
| 5.) 4-Guanidinobenzoesäure-(3-methoxycarbonylphenyl)-ester Hydrochlorid | 550 |
| 6.) 4-Guanidinobenzoesäure-(3-carbamoylphenyl)-ester Hydrochlorid | 320 |

## Tabelle II

Die Hemmbarkeit von Humanakrosin durch Aryl-4-Guanidinobenzoatderivate

Gelatinolyse

| | 100 µg/ml | 10 µg/ml | 1 µg/ml |
|---|---|---|---|
| 1.) 4-Guanidinobenzoesäure-(2-methoxycarbonylphenyl)-ester Hydrochlorid | ∅ | + bis ++ | ++ bis +++ |
| 2.) 4-Guanidinobenzoesäure-(2-cyanophenyl)-ester Hydrochlorid | ∅ | ∅ | ++ |
| 3.) 4-Guanidinobenzoesäure-(3-methoxycarbonylphenyl)-ester Hydrochlorid | ∅ | ∅ bis ++ | ++ |
| 4.) 4-Guanidinobenzoesäure-(3-ethoxycarbonylphenyl)-ester Hydrochlorid | ∅ | + bis ++ | ++ bis +++ |

∅ = vollständige )
+ = starke ) Hemmung der Akrosinaktivität
++ = mäßige )
+++ = keine )

**T a b e l l e   III**

**Die Beeinflussung der in vitro-Fertilisation durch verschiedene Akrosininhibitoren**

| Verbindung | ug/ml | Anzahl Oocyten | Anzahl 2-Zellen-Embryos | % Fertilisation |
|---|---|---|---|---|
| 1.) 4-Guanidinobenzoesäure-(2-methoxycarbonylphenyl)-ester Hydrochlorid | 50 | 605 | 6 | 1 |
| | 25 | 257 | 114 | 44 |
| | 5 | 250 | 195 | 78 |
| | Kontrolle + 0,1 % DMSO | 184 | 139 | 76 |
| 2.) 4-Guanidinobenzoesäure-(2-cyanophenyl)-ester Hydrochlorid | 50 | 301 | 22 | 7 |
| | 25 | 89 | 24 | 27 |
| | 5 | 227 | 134 | 59 |
| 3.) 4-Guanidinobenzoesäure-(3-methoxycarbonylphenyl)-ester Hydrochlorid | 50 | 118 | 4 | 3 |
| | 25 | 114 | 16 | 13 |
| | 5 | 136 | 45 | 33 |
| | Kontrolle HCL | 343 | 239 | 70 |

**T a b e l l e IV**

**Der Einfluß verschiedener Akrosininhibitoren auf die in-vivo-Fertilisation beim Kaninchen**

| Verbindung | | Anzahl Oocyten | geteilt | nicht geteilt | % geteilt |
|---|---|---|---|---|---|
| | Polyethylenglykel (PEG) *) als Kontrolle | 310 | 186 | 124 | 60 |
| 1.) 4-Guanidinobenzoesäure-(2-methoxycarbonyl-phenyl)-ester Hydrochlorid | 500 µg/ml | 171 | 88 | 83 | 51 |
| 2.) 4-Guanidinobenzoesäure-(2-cyanophenyl)-ester Hydrochlorid | 500 µg/ml | 160 | 2 | 158 | 1 |
| 3.) 4-Guanidinobenzoesäure-(3-methoxycarbonyl-phenyl)-ester Hydrochlorid | 500 µg/ml | 320 | 12 | 308 | 4 |

*) Zusammensetzung des PEG: 70 % PEG 6000 (10 % Lösung) + 30 % PEG 300 } 1:4 Verdünnung

Beispiel 1

Eine Mischung aus 1,0 g (4,64 mmol) 4-Guanidinobenzoesäure Hydrochlorid, 2,12 g (13,92 mmol) 3-Hydroxybenzoesäuremethylester und 100 mg (0,58 mmol) wasserfreier p-Toluolsulfonsäure wird unter Argon bei 0 °C in 4 ml Pyridin 15 Stunden gerührt. Nach Zugabe von 2,87 g (13,92 mmol) Dicyclohexylcarbodiimid wird noch 3 Tage bei Raumtemperatur gerührt und anschließend mit 1 ml Eisessig bei 0 °C versetzt. Nach einer Rührzeit von 2 Stunden bei 0 °C wird vom Feststoff abfiltriert, das Filtrat mit ca. 25 g Eis versetzt und mit 5 N Salzsäure bis pH = 1 angesäuert. Nach Unterschichtung mit 25 ml Methylenchlorid wird noch 1 Stunde unter Eiskühlung gerührt und danach bei 50 °C eingeengt. Das anfallende Öl-Kristall-Gemisch (9,93 g) wird an Kieselgel mit Methylenchlorid/Methanol/Eisessig (80:20:0,1) chromatographiert. Anschließende Umkristallisation aus Chloroform liefert 270 mg 4-Guanidinobenzoesäure-(3-methoxycarbonylphenyl)-ester Hydrochlorid vom Schmelzpunkt 194 - 195 °C.

Beispiel 2

Durchführung und eingesetzte Mengen entsprechen denen des Beispiels 1. Jedoch werden anstelle des 3-Hydroxybenzoesäuremethylesters 2,31 g (13,92 mmol) 3-Hydroxybenzoesäureethylester verwendet. Das nach dem Einengen anfallende Öl-Kristall-Gemisch (12,4 g) wird an Kieselgel mit Methylenchlorid/Methanol/Eisessig (80:10:0,1) chromatographiert und die entsprechende Fraktion (960 mg) aus Aceton/Äthanol umkristallisiert. Es werden 330 mg 4-Guanidinobenzoesäure-(3-ethoxycarbonylphenyl)-ester Hydrochlorid vom Schmelzpunkt 137 °C erhalten.

Beispiel 3

Durchführung und eingesetzte Mengen entsprechen denen des Beispiels 1. Jedoch werden anstelle des 3-Hydroxybenzoesäuremethylesters 1,66 g (13,92 mmol) 2-Hydroxybenzonitril verwendet. Das nach dem Einengen erhaltene Öl-Kristall-Gemisch (12,55 g) wird an Kieselgel mit Methylenchlorid/Methanol/Eisessig (90:10:0,1) chromatographiert und die entsprechende Fraktion aus 15 ml Chloroform umkristallisiert. Es werden 960 mg 4-Guanidinobenzoesäure-(2-cyanphenyl)-ester Hydrochlorid vom Schmelzpunkt 178 °C erhalten.

Beispiel 4

Durchführung und eingesetzte Mengen entsprechen denen des Beispiels 1. Jedoch werden anstelle des 3-Hydroxybenzoesäuremethylesters 2,15 g (13,92 mmol) 2,3-Dimethoxyphenol verwendet. Das nach dem Einengen erhaltene Öl (4,46 g) wird an Kieselgel mit Methylenchlorid/Methanol/Eisessig (8:2:1) chromatographiert und die entsprechende Fraktion von 750 mg nacheinander mit 70 ml Ethanol/Methylenchlorid (1:1) und 10 ml Methylenchlorid bei Raumtemperatur digeriert. Nach dem Absaugen werden 245 mg 4-Guanidinobenzoesäure-(2,3-dimethoxyphenyl)-ester Hydrochlorid vom Schmelzpunkt 197 - 201 °C (Zersetzung) erhalten.

Beispiel 5

Durchführung und eingesetzte Mengen entsprechen denen des Beispiels 1. Jedoch werden anstelle des 3-Hydroxybenzoesäuremethylesters 1,91 g (13,92 mmol) 3-Hydroxybenzamid verwendet. Nach Behandlung mit 5 N Salzsäure und Unterschichtung mit Methylenchlorid wird der ausgefallene

Feststoff (820 mg) abgesaugt und mit 17 ml Chloroform ausgekocht. Durch Filtration werden 530 mg 4-Guanidinobenzoesäure-(3-carbamoylphenyl)-ester Hydrochlorid vom Schmelzpunkt 104 - 105 °C erhalten.

## Patentansprüche

1.) Guanidinobenzoesäureester der allgemeinen Formel I

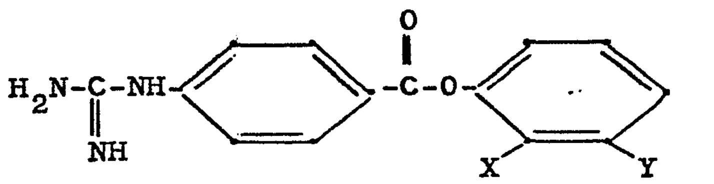

(I),

worin

X für ein Wasserstoffatom, einen Cyanrest oder eine Alkoxygruppe mit 1 - 4 Kohlenstoffatomen und

Y für ein Wasserstoffatom, eine Alkoxy- oder Alkoxycarbonylgruppe mit 1 - 4 Kohlenstoffatomen oder eine $-\underset{\overset{\|}{O}}{C}-NRR'$-Gruppe mit R und R' in der Bedeutung eines Wasserstoffatoms oder eines niederen gesättigten Alkylrestes mit 1 - 4 Kohlenstoffatomen steht, wobei X und Y nicht gleichzeitig Wasserstoff sein sollen, sowie deren physiologisch unbedenkliche Salze mit anorganischen oder organischen Säuren.

2.) 4-Guanidinobenzoesäure-(3-methoxycarbonylphenyl)-ester und dessen Hydrochlorid.

3.) 4-Guanidinobenzoesäure-(3-ethoxycarbonylphenyl)-ester und dessen Hydrochlorid.

4.) 4-Guanidinobenzoesäure-(2-cyanphenyl)-ester und dessen Hydrochlord.

0170180

5.) 4-Guanidinobenzoesäure-(2,3-dimethoxyphenyl)-ester und dessen Hydrochlorid.

6.) 4-Guanidinobenzoesäure-(3-carbamoylphenyl)-ester und dessen Hydrochlorid.

7.) Verfahren zur Herstellung von Verbindungen der allgemeinen Formel I, dadurch gekennzeichnet, daß man in an sich bekannter Weise ein Salz der 4-Guanidinobenzoesäure mit einem Phenol der allgemeinen Formel II

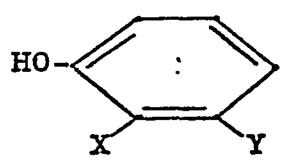

(II),

wobei X und Y die in Anspruch 1 genannte Bedeutung haben, kondensiert.

8.) Arzneimittel auf Basis einer oder mehrerer Verbindung(en) gemäß den Ansprüchen 1 - 6.

9.) Verwendung einer oder mehrerer Verbindung(en) gemäß den Ansprüchen 1 - 6 zur Herstellung von Arzneimitteln für die vaginale Kontrazeption.

Europäisches Patentamt

**0170180**

Nummer der Anmeldung

# EUROPÄISCHER RECHERCHENBERICHT

| EINSCHLÄGIGE DOKUMENTE | | | EP 85109112.4 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
| D,X | US - A - 4 423 069 (JOANNE M. KAMINSKI et al.)<br>* Spalten 1,2 *<br>-- | 1,7-9 | C 07 C 129/12<br>A 61 K 31/245 |
| A | US - A - 4 283 418 (SETSURO FUJII et al.)<br>* Spalten 1,2 *<br>-- | 1,7,8 | |
| A | DE - A1 - 3 031 791 (ONO PHARMACEUTICAL CO. LTD.)<br>* Ansprüche 1,6,7 *<br>-- | 1,7,8 | |
| A | PATENT ABSTRACTS OF JAPAN, unexamined applications, C Field, Bd. 5, Nr. 188, 27. November 1981<br>THE PATENT OFFICE JAPANESE GOVERNMENT<br>Seite 25 C 81<br>* Kokai-Nr. 56-110 664 (TORII YAKUHIN) *<br>---- | 1,7,8 | RECHERCHIERTE SACHGEBIETE (Int. Cl 4)<br>C 07 C 129/00 |

Der vorliegende Recherchenbericht wurde für alle Patentansprüche erstellt

| Recherchenort | Abschlußdatum der Recherche | Prüfer |
|---|---|---|
| WIEN | 09-10-1985 | REIF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82